(19) Europäisches Patentamt / European Patent Office / Office européen des brevets

(11) **EP 4 112 598 B1**

(12) **EUROPEAN PATENT SPECIFICATION**

(45) Date of publication and mention
of the grant of the patent:
**18.12.2024 Bulletin 2024/51**

(21) Application number: **21759902.6**

(22) Date of filing: **26.02.2021**

(51) International Patent Classification (IPC):
*C07C 253/28* (2006.01)  *C07C 253/34* (2006.01)
*C07C 255/51* (2006.01)

(52) Cooperative Patent Classification (CPC):
(C-Sets available)
**C07C 253/28; C07C 253/34**       (Cont.)

(86) International application number:
**PCT/JP2021/007291**

(87) International publication number:
**WO 2021/172499 (02.09.2021 Gazette 2021/35)**

(54) **METHOD FOR PRODUCING PURIFIED PHTHALONITRILE AND METHOD FOR PURIFYING PHTHALONITRILE**

VERFAHREN ZUR HERSTELLUNG VON GEREINIGTEM PHTHALONITRIL UND VERFAHREN ZUR REINIGUNG VON PHTHALODINITRIL

PROCÉDÉ DE PRODUCTION DE PHTALONITRILE PURIFIÉ ET PROCÉDÉ DE PURIFICATION DE PHTALONITRILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2020 JP 2020033525**

(43) Date of publication of application:
**04.01.2023 Bulletin 2023/01**

(73) Proprietor: **MITSUBISHI GAS CHEMICAL COMPANY, INC.**
**Chiyoda-ku**
**Tokyo 100-8324 (JP)**

(72) Inventor: **MUNEYASU, Kuniaki**
**Niigata-shi, Niigata 950-3121 (JP)**

(74) Representative: **Hoffmann Eitle**
**Patent- und Rechtsanwälte PartmbB**
**Arabellastraße 30**
**81925 München (DE)**

(56) References cited:
**CN-A- 110 132 242    CN-A- 110 902 145
JP-A- 2002 097 177    JP-A- H02 247 285
JP-A- H08 206 643     JP-A- S48 023 727
JP-A- S48 024 964     JP-A- S5 416 445
US-A- 5 045 156       US-A1- 2002 035 287**

(52) Cooperative Patent Classification (CPC): (Cont.)

C-Sets
**C07C 253/28, C07C 255/51;
C07C 253/34, C07C 255/51**

C-Sets
**C07C 253/28, C07C 255/51;
C07C 253/34, C07C 255/51**

**Description**

TECHNICAL FIELD

The present invention relates to a method for producing a purified phthalonitrile and a method for purifying a phthalonitrile. More specifically, the present invention relates to a method for producing a purified phthalonitrile and a method for purifying a phthalonitrile, wherein a phthalonitrile obtained by an ammoxidation reaction is purified.

BACKGROUND ART

A method of reacting, in the presence of a catalyst, an organic-substituent-containing carbon-ring or heterocyclic compound with ammonia and an oxygen-containing gas is called ammoxidation. For an ammoxidation reaction, a gas-phase catalytic fluidized reaction is generally employed. By the ammoxidation reaction, a nitrile compound such as a phthalonitrile is produced. Phthalonitriles are useful as raw materials for producing synthetic resins, agricultural chemicals, etc., and as intermediate raw materials for amines, isocyanates, etc. Various methods for separating a phthalonitrile from a reaction product gas generated by an ammoxidation reaction have been disclosed.

For example, Non-Patent Document 1 discloses a method in which: isophthalonitrile in a reaction product gas is collected by using an organic solvent; then a collection liquid is supplied to a solvent recovery column, the solvent is removed from the column top and crude isophthalonitrile is collected from the column bottom; and then said crude isophthalonitrile is supplied to a purification column and purified isophthalonitrile is collected from the column top. However, when using the method of Non-Patent Document 1, the phthalonitrile is easily lost.

Further, Patent Document 1 discloses a method for producing isophthalonitrile, in which: isophthalonitrile in a reaction product gas generated by an ammoxidation reaction is collected by using an organic solvent; then a high boiling point impurity is separated in the first distillation step; and the organic solvent is volatilized and separated in the second distillation step, thereby obtaining isophthalonitrile. However, there is a room for improvement from the viewpoint of stably obtaining a high-purity phthalonitrile with high yield for a long period of time. Patent document 2 discloses a method for purifying isophthalonitrile, including separating isophthalonitrile from a gas produced by reacting m-xylene with ammonia and oxygen-containing gas in the presence of a catalyst comprising a) a trapping step for bringing the gas into contact with an organic solvent having a boiling point lower than that of isophthalonitrile; b) a high-boiling-point impurity separation step for distilling a liquid in which isophthalonitrile is trapped in the trapping step, to thereby recover isophthalonitrile and the organic solvent from the top of the column and separate at the bottom of the column impurities having boiling points higher than that of isophthalonitrile; and c) a rectification step for subjecting isophthalonitrile and the organic solvent resulting from the high-boiling-point impurity separation step to rectification, to thereby recover the organic solvent from the top of the column and recover liquefied isophthalonitrile of high purity at the bottom of the column.

PRIOR ART DOCUMENTS

PATENT DOCUMENTS

Patent Document 1: Japanese Patent No. 4929523; Patent Document 2: US2002/035287A1

NON-PATENT DOCUMENTS

Non-Patent Document 1: The Japan Petroleum Institute Ed., Process Handbook (published in 1976) MGC-Badger Isophthalonitril Process

SUMMARY OF THE INVENTION

PROBLEMS TO BE SOLVED BY THE INVENTION

It has been desired to develop a method for producing a purified phthalonitrile and a method for purifying a phthalonitrile, by which a phthalonitrile can be stably obtained for a long period of time.

Further, it has been desired to establish a method for treating a treated liquid containing a by-product, which is for stably obtaining a phthalonitrile by means of an ammoxidation reaction for a long period of time.

Moreover, it has been desired to develop a method for producing a purified phthalonitrile and a method for purifying a phthalonitrile, by which a high-purity phthalonitrile can be stably obtained with high yield for a long period of time.

MEANS FOR SOLVING THE PROBLEMS

The present inventors diligently made researches on a method for obtaining a purified phthalonitrile from a mixed gas of a phthalonitrile and impurities generated by an ammoxidation reaction. As a result, the present inventors found that, by separating a phthalonitrile from an organic solvent, etc. after removing a high boiling point impurity, and by collecting and burning the high boiling point impurity kept in a liquid state, change in quality of a bottom liquid is prevented, thereby stably obtaining a phthalonitrile for a long period of time, and found that, preferably, solidification and closure due to change in quality of a bottom liquid is prevented while the loss of a phthalonitrile due to change in quality thereof is reduced, thereby stably obtaining a high-purity phthalonitrile with high yield for a long period of time. Thus the present invention was achieved.

The present invention provides:

[1] A method for producing a purified phthalonitrile, which comprises:

a production reaction step in which a reaction product gas containing a phthalonitrile and a cyanobenzamide is obtained by reacting ammonia, oxygen and xylene in the presence of a catalyst;
a collection step in which a collection liquid is obtained by bringing the reaction product gas into contact with an organic solvent that has a boiling point lower than that of the phthalonitrile;
a separation step in which the collection liquid is distilled by a high boiling point fraction-separating column, thereby obtaining a gas that contains the phthalonitrile and the organic solvent from the column top, while obtaining a bottom liquid that contains a cyanobenzamide from the column bottom, said bottom liquid having a phthalonitrile content of 90% by mass or less;
a combustion step in which the obtained bottom liquid is subjected to combustion, while being kept in a liquid state; and
a rectification step in which a purified phthalonitrile is obtained by removing the organic solvent from the gas obtained from the column top, wherein in the separation step, the liquid residence time at the column bottom is adjusted to 72 hours or less, the distillation pressure is adjusted to 12 kPa or less, and the temperature of the column bottom is adjusted to 200 to 230°C.

[2] The method for producing a purified phthalonitrile according to item [1] wherein:

the steps from the production reaction step to the rectification step are continuously carried out;
in the production reaction step, the reaction product gas further contains a tolunitrile; and
the tolunitrile is recovered and used as the organic solvent in the collection step.

[3] The method for producing a purified phthalonitrile according to item [2], wherein the tolunitrile is meta-tolunitrile, para-tolunitrile or a mixture thereof.
[4] The method for producing a purified phthalonitrile according to any one of items [1] to [3], wherein the cyanobenzamide is 3-cyanobenzamide, 4-cyanobenzamide or a mixture thereof.
[5] The method for producing a purified phthalonitrile according to any one of items [1] to [4], wherein the phthalonitrile is isophthalonitrile, terephthalonitrile or a mixture thereof.
[6] A method for purifying a phthalonitrile, which comprises:

a collection step in which a collection liquid is obtained by bringing a reaction product gas that contains a phthalonitrile and a cyanobenzamide into contact with an organic solvent that has a boiling point lower than that of the phthalonitrile;
a separation step in which the collection liquid is distilled by a high boiling point fraction-separating column, thereby obtaining a gas that contains the phthalonitrile and the organic solvent from the column top, while obtaining a bottom liquid that contains a cyanobenzamide from the column bottom, said bottom liquid having a phthalonitrile content of 90% by mass or less;
a combustion step in which the obtained bottom liquid is subjected to combustion, while being kept in a liquid state; and
a rectification step in which a purified phthalonitrile is obtained by removing the organic solvent from the gas obtained from the column top, wherein in the separation step, the liquid residence time at the column bottom is adjusted to 72 hours or less, the distillation pressure is adjusted to 12 kPa or less, and the temperature of the column bottom is adjusted to 200 to 230°C.

[7] The method for producing a purified phthalonitrile according to any one of items [1] to [5], wherein the combustion step further includes generating steam by utilizing heat generated by combustion.

[8] The method for producing a purified phthalonitrile according to item [7], wherein the combustion step further includes transferring the bottom liquid by using a piping, and wherein the temperature of the piping is kept by using the steam.

[9] The method for producing a purified phthalonitrile according to item [7] or [8], wherein the steam is used as a heat source in the collection step, the separation step or the rectification step.

ADVANTAGEOUS EFFECT OF THE INVENTION

The present invention provides a method for producing a purified phthalonitrile and a method for purifying a phthalonitrile, by which a phthalonitrile can be stably obtained for a long period of time.

Further, the present invention provides a method for treating a treated liquid containing a by-product, which is for stably obtaining a phthalonitrile by means of an ammoxidation reaction for a long period of time.

Moreover, the present invention provides a method for producing a purified phthalonitrile and a method for purifying a phthalonitrile, by which a high-purity phthalonitrile can be stably obtained with high yield for a long period of time.

BRIEF DESCRIPTION OF THE DRAWINGS

FIG. 1 is a flow diagram showing an example of the production method of the present invention.
FIG. 2 shows the flow of Experiment Examples.
FIG. 3 is a flow diagram showing another example of the production method of the present invention.

EMBODIMENTS FOR CARRYING OUT THE INVENTION

<Method for producing purified phthalonitrile>

One embodiment of the present invention relates to a method for producing a purified phthalonitrile. The production method of the present invention includes: (1) a production reaction step in which a reaction product gas containing a phthalonitrile and an impurity is obtained by means of an ammoxidation reaction; (2) a collection step in which a collection liquid is obtained by bringing the reaction product gas into contact with an organic solvent; (3) a separation step in which the obtained collection liquid is distilled by a high boiling point fraction-separating column, thereby obtaining a gas that contains the phthalonitrile and the organic solvent from the column top, while collecting a bottom liquid that contains a high boiling point impurity such as a cyanobenzamide from the column bottom; (4) a combustion step in which the collected bottom liquid is subjected to combustion, while being kept in a liquid state; and (5) a rectification step in which a purified phthalonitrile is obtained by removing the organic solvent and the like from the gas obtained from the column top, wherein in the separation step, the liquid residence time at the column bottom is adjusted to 72 hours or less, the distillation pressure is adjusted to 12 kPa or less, and the temperature of the column bottom is adjusted to 200 to 230°C.

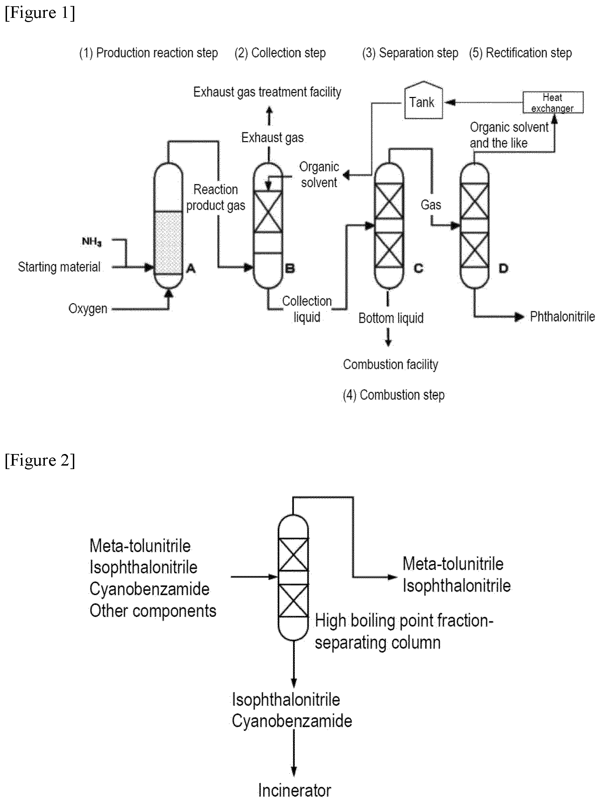

Hereinafter, the production method of the present invention will be described with reference to Figure 1, but the production method of the present invention is not limited to the embodiment shown in Figure 1.

(1) Production reaction step

In the production reaction step, an ammoxidation reaction, in which xylene, ammonia and oxygen are reacted, is performed. In Figure 1, the production reaction step is carried out in an ammoxidation reactor represented by A.

Since the ammoxidation reaction has a large heat of reaction, it is preferred to employ a gas phase fluidized bed reaction in order to obtain a uniform temperature distribution in the reactor. As the reactor, various types of fluidized bed reactors can be used.

For the ammoxidation reaction, a publicly-known catalyst such as a catalyst containing a V-Cr-B-Mo-based oxide described in Japanese Laid-Open Patent Publication No. H11-209332 and a catalyst containing a Fe-Sb-V-based oxide described in Japanese Laid-Open Patent Publication No. H09-71561 can be used.

Oxygen is generally supplied by supplying an oxygen-containing gas in which oxygen and other gases coexist, for example, air, to the reactor. As the oxygen-containing gas, a gas obtained by further enriching oxygen in air may be used. Further, a diluent such as nitrogen and carbon dioxide gas can be used in combination. The amount of oxygen to be used is preferably 3 times or more, and more preferably 4 to 100 times relative to 1 mol of xylene. When the amount is excessively small, the yield of the phthalonitrile may be reduced. When the amount is excessively large, the space time yield may be reduced.

Examples of the xylene include ortho-xylene, meta-xylene and para-xylene, but meta-xylene is preferred. When ammoxidation is performed using air, the concentration of the xylene in a raw material gas supplied to the reactor is preferably 0.2 to 10% by volume, and more preferably 0.5 to 5% by volume. When the concentration of the xylene is excessively high, the yield of the phthalonitrile may be reduced. When the concentration of the xylene is excessively low, the space time yield may be reduced.

As the ammonia, an industrial grade ammonia can be used. The amount of the ammonia to be used is preferably 2 to 20 times, and more preferably 6 to 15 times the molar quantity of the xylene. When the amount is excessively small, the yield of the phthalonitrile may be reduced. When the amount is excessively large, the space time yield may be reduced.

As shown in Figure 1, the ammonia can be mixed with the xylene to be supplied to the reactor. Further, under conditions for avoiding the explosion range (combustible range), a portion of the oxygen-containing gas can be further mixed with a mixed gas of the ammonia and the xylene to be supplied.

The reaction temperature for ammoxidation is preferably 300 to 500°C, and more preferably 330 to 470°C. When the reaction temperature is excessively low, the conversion rate may be reduced. When the reaction temperature is excessively high, the yield of the phthalonitrile may be reduced due to increase of by-products such as carbon dioxide gas and hydrogen cyanide.

The reaction pressure may be either ordinary pressure, elevated pressure or reduced pressure, but a range of from ordinary pressure (atmospheric pressure; usually an absolute pressure of 0.1013 MPa) to 0.3 Mpa is preferred. The reaction pressure is preferably 0.2 to 0.3 Mpa.

When using a catalyst, the contact time between a reaction gas and the catalyst depends on conditions including the feed molar ratio of the ammonia and the oxygen-containing gas to the xylene and the reaction temperature, but it is usually 0.3 to 30 seconds.

In the production reaction step (1), a reaction product gas containing a phthalonitrile and impurities is obtained.

Examples of the phthalonitrile include isophthalonitrile, terephthalonitrile and a mixture thereof.

Among the impurities contained in the reaction gas, examples of a high boiling point impurity having a boiling point higher than that of the phthalonitrile, preferably isophthalonitrile include a cyanobenzamide. Examples of the cyanobenzamide include 3-cyanobenzamide, 4-cyanobenzamide and a mixture thereof. Other than the cyanobenzamide, meta-toluamide and 3-cyanobenzoic acid may be contained.

The reaction product gas may contain a low boiling point impurity having a boiling point lower than that of the phthalonitrile, preferably isophthalonitrile. Examples of the low boiling point impurity include a tolunitrile. Examples of the tolunitrile include meta-tolunitrile, para-tolunitrile and a mixture thereof. A low boiling point impurity other than the tolunitrile may be contained.

In the case where the low boiling point impurity such as the tolunitrile is by-produced, it is preferred that the steps from the production reaction step (1) to the rectification step (5) are continuously carried out, and that the by-produced low boiling point impurity such as the tolunitrile is recovered in the production reaction step (1) and used as an organic solvent in the collection step (2).


(2) Collection step

Next, a collection liquid is obtained by bringing the reaction product gas into contact with an organic solvent. In Figure 1, the collection step (2) is carried out in a collection column represented by B. Specifically, the reaction product gas from the ammoxidation reactor A is introduced into the collection column B and brought into contact with the organic solvent. As shown in Figure 1, an absorption part consisting of a tray or packed bed may be provided to the upper part of the collection column. In this case, the organic solvent is supplied from the upper part of the collection column.

As the organic solvent, one having a boiling point lower than that of the phthalonitrile is used. At least one selected from the group consisting of an alkylbenzene, a heterocyclic compound, an aromatic nitrile compound and a heterocyclic nitrile compound is preferred. More preferred is an organic solvent that is at least one selected from the group consisting of an alkylbenzene, a heterocyclic compound, an aromatic nitrile compound and a heterocyclic nitrile compound, wherein the solubility of the phthalonitrile is high, and wherein the organic solvent is inactive to the phthalonitrile.

Specific examples of the organic solvent include meta-xylene, pseudocumene, mesitylene, ethylbenzene, methylpyridine, benzonitrile, meta-tolunitrile, para-tolunitrile and cyanopyridine. These organic solvents can be used solely, or two or more of them can be used in combination. Meta-tolunitrile is particularly preferred.

As described above, the low boiling point impurity generated in the production reaction step (1) may be utilized as the organic solvent.

The temperature of the collection column is set in a manner such that the temperature of the collection liquid collected at the column bottom (sometimes referred to as "the liquid phase portion of the column bottom") becomes equal to or lower than the boiling point. Specifically, in the collection step (2), the composition of the liquid phase portion of the column bottom depends on the amount of the reaction product gas from the ammoxidation reactor A and the amount of the organic solvent supplied to the collection column B, and in this regard, the temperature of the column bottom of the collection column is set in a manner such that the temperature of the liquid phase portion of the column bottom with a composition in each case becomes equal to or lower than the boiling point.

The pressure in the collection column may be either ordinary pressure, elevated pressure or reduced pressure, but is usually set within the range of from ordinary pressure to ammoxidation reaction pressure.

The high boiling point impurity other than the cyanobenzamide, such as meta-toluamide and 3-cyanobenzoic acid, may be generated in the collection step (2) in addition to the production reaction step (1).

In the collection step (2), the reaction product gas is injected into the liquid phase portion of the column bottom of the collection column. By this, the phthalonitrile in the reaction product gas is dissolved in the organic solvent and collected together with the high boiling point impurity such as the cyanobenzamide, and optionally the low boiling point impurity. Meanwhile, unreacted ammonia, a hydrophilic by-product such as hydrogen cyanide, carbon dioxide gas, water, carbon monoxide, nitrogen, oxygen, etc. are not collected by the organic solvent and are separated as exhaust gas. The exhaust gas is discharged from the upper part of the collection column. The collection liquid is drawn out from the column bottom of the collection column and provided to the subsequent separation step (3).

(3) Separation step

In the separation step, the high boiling point impurity is removed from the collection liquid. Specifically, the collection liquid is distilled in a high boiling point fraction-separating column (represented by C in Figure 1), and from the column top, a gas that contains the phthalonitrile and the organic solvent and optionally contains the low boiling point impurity is obtained, while a bottom liquid that contains the high boiling point impurity is collected from the column bottom, wherein the liquid residence time at the column bottom is adjusted to 72 hours or less, the distillation pressure is adjusted to 12 kPa or less, and the temperature of the column bottom is adjusted to 200 to 230°C.

In the separation step (3), a distillation apparatus such as a packed column, a tray column and a flash drum can be used. Further, the separation step (3) can be carried out under reduced pressure using a batch system or continuous system.

The bottom liquid may contain the phthalonitrile. Specifically, the content of the phthalonitrile is 90% by mass or less. The content of the phthalonitrile is preferably 60% by mass or less, more preferably 40% by mass or less, even more preferably 20% by mass or less, particularly preferably 10% by mass or less, and most preferably less than 10% by mass. The lower limit of the content of the phthalonitrile is not particularly limited, but it is preferably 5% by mass or more.

As the method for measuring the content, gas chromatography is preferred. The content is preferably measured after the separation step is completed.

The bottom liquid is collected while it is kept in a liquid state.

The bottom liquid may contain the high boiling point impurity such as the cyanobenzamide and the phthalonitrile, and in this regard, under the coexistence of impurities such as the cyanobenzamide, an ammoxidation catalyst and a metal, the phthalonitrile is unstable against heat and change in quality such as amidation and polymerization is easily caused. The longer the time for exposure to high temperature is, and the higher the temperature to be exposed is, the more easily change in quality is caused, and the degree thereof is increased. For this reason, for the purpose of carrying out the separation step (3) quickly and at a temperature as low as possible, it is preferred to control conditions with respect to the high boiling point fraction-separating column, specifically, the column bottom temperature, the residence time of the bottom liquid at the column bottom and the distillation pressure. By doing this, change in quality of the phthalonitrile can be suppressed, increase in melting points of components contained in the bottom liquid can be suppressed, and increase in the viscosity of the bottom liquid can be suppressed. It is considered that the loss of the phthalonitrile due to change in quality thereof can be suppressed thereby. Further, it is considered that solidification and closure due to change in quality of the bottom liquid can be prevented thereby.

Specifically, the distillation pressure of the bottom liquid is preferably 12 kPa or less, more preferably less than 12 kPa, and particularly preferably less than 8 kPa. The lower limit of the distillation pressure is not particularly limited, but it is preferably 5 kPa or more.

The temperature of the bottom liquid is preferably 200 to 230°C, more preferably 210 to 230°C, and particularly preferably 220 to 230°C. When the temperature of the bottom liquid is lower than 200°C, since it is lower than the melting point of the high boiling point impurity such as the cyanobenzamide, the bottom liquid may be solidified.

The liquid residence time of the bottom liquid is preferably less than 96 hours, and particularly preferably 72 hours or less. The lower limit of the liquid residence time is not particularly limited and it is sufficient when it is 0 hour or more, but it is preferably 12 hours or more, particularly preferably 24 hours or more, and most preferably 48 hours or more.

Regarding the bottom liquid, it is preferred that the distillation pressure is 5 to 12 kPa, that the temperature is 200 to 230°C, and that the liquid residence time is 12 to 72 hours. It is more preferred that the distillation pressure is 5 kPa or more but less than 12 kPa, that the temperature is 210 to 230°C, and that the liquid residence time is 12 to 72 hours. It is particularly preferred that the distillation pressure is 5 kPa or more but less than 8 kPa, that the temperature is 220 to 230°C, and that the liquid residence time is 12 to 72 hours.

The liquid residence time can be calculated according to the below-described formula.

$$X=B/A$$

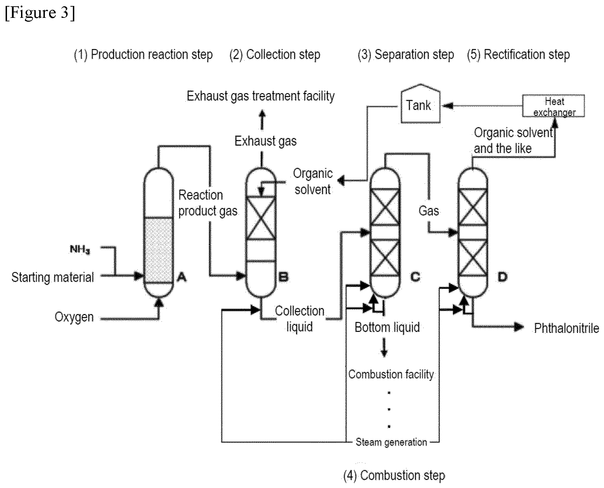

In the formula, X (Hr) represents the liquid residence time, A ($m^3$/Hr) represents the discharge amount of the bottom liquid, and B ($m^3$) represent the amount of the liquid held at the column bottom of the high boiling point fraction-separating column. As shown in Figure 3, in the case where steam is generated using heat generated by combustion and said steam is supplied to the high boiling point fraction-separating column, B represents the amount of the liquid held at the column bottom of the high boiling point fraction-separating column, in a piping for circulating the bottom liquid extending from the column bottom, and in a reboiler.

The bottom liquid collected from the column bottom is sent to the combustion step (4). Meanwhile, the gas collected from the column top is sent to the rectification step (5).

(4) Combustion step

The obtained bottom liquid is provided to the combustion step (4). In the present invention, when the bottom liquid is transferred to an incinerator and subjected to combustion, solidification and vaporization of the bottom liquid is avoided as much as possible and the bottom liquid is kept in a liquid state.

During transferring to the incinerator, the bottom liquid is kept at a temperature that is higher than the melting point and lower than the boiling point. As the means for transferring the bottom liquid with its temperature being kept within this range, a publicly-known means, for example, heating a piping through which the bottom liquid is passed using a heater or the like, may be utilized. Preferably, the bottom liquid is passed through the inside passage of a double pipe, and a fluid for keeping temperature such as steam is passed though the outside passage of the double pipe.

For transferring the bottom liquid and supplying it to the incinerator, a pump is preferably used.

As the incinerator, a publicly-known one can be used. For example, a horizontal cylindrical furnace, a vertical cylindrical furnace or the like can be used. Preferably, the bottom liquid kept in a liquid state is injected into the incinerator using a nozzle and subjected to combustion.

The heat generated by combustion can be utilized for the generation of steam. For example, a water drum, a pipe through which water is passed (hereinafter referred to as "the water pipe") and a steam drum are provided to the incinerator for combustion of the bottom liquid (boiler), and water in the water pipe is heated by the heat generated by combustion, thereby generating steam. The generated steam is sent to the outside of the incinerator via a piping for steam which is kept hot.

The generated steam can be utilized for various applications. For example, a piping for steam is arranged between a combustion facility and a piping for the bottom liquid, and steam is sent to the piping for the bottom liquid, thereby keeping the temperature of the bottom liquid during transferring. As the piping for the bottom liquid, a double piping is used. Preferably, as shown in Figure 3, the piping for the bottom liquid has a structure in which the bottom liquid is circulated while a part thereof is discharged, a reboiler (also referred to as "heat exchanger"; omitted in Figure 3) is provided to the circulation portion of the piping for the bottom liquid, and steam is supplied to the reboiler.

Further, by arranging a piping for steam between the combustion facility and a piping for the collection liquid extending from the collection column B, the high boiling point fraction-separating column C or the rectification column D, steam can be utilized as a heat source in the collection step (2), the separation step (3) or the rectification step (5).

Moreover, by arranging a piping for steam to a piping for a purified phthalonitrile extending from the rectification column D, steam can be utilized as a heat source for preventing the purified phthalonitrile from having a temperature equal to or lower than the melting point and solidifying. As the piping for the purified phthalonitrile, a double piping is used. Preferably, as shown in Figure 3, the piping for the purified phthalonitrile has a structure in which the purified phthalonitrile is circulated while a part thereof is discharged, a reboiler (also referred to as "heat exchanger"; omitted in Figure 3) is provided to the circulation portion of the piping for the purified phthalonitrile, and steam is supplied to the reboiler.

(5) Rectification step

Meanwhile, referring to Figure 1, the gas obtained from the column top in the separation step (3) contains not only the phthalonitrile of interest, but also other components such as the organic solvent and the low boiling point impurity. For this reason, in the rectification step (5), the phthalonitrile is separated from the other components. Specifically, distillation is carried out in the rectification column (represented by D in Figure 1), the other components are removed from the column top, and the purified phthalonitrile of interest in a liquid state is collected from the column bottom.

When providing the gas obtained in the separation step (3) to the rectification step (5), the phthalonitrile and the other components such as the organic solvent may be provided in a gas state, or may be provided after being condensed into a liquid state. These are preferably provided in a gas state because energy consumption can be reduced in this case.

The operating pressure of the rectification column is preferably reduced pressure. Specifically, as the operating pressure of the rectification column, a high vacuum condition within a range in which the phthalonitrile is not precipitated in the column is selected. For example, it is desirable that the pressure of the rectification column in the case of using meta-tolunitrile as the organic solvent is 5 to 10 kPa. When the condition in which the phthalonitrile is brought into contact with a sufficient amount of the organic solvent in the rectification column, and the condition in which the main component in a condensation part is a solvent; the temperature thereof is low; there is no vapor pressure of the phthalonitrile in the condensation part; and the phthalonitrile is not scattered over a vacuum exhaust system, are satisfied, a scrubber between the condensation part and the vacuum exhaust part may be unnecessary.

When the tolunitrile is included in the other components, the separated and collected tolunitrile is preferably reused as the organic solvent in the collection step (2). For reuse, for example, as shown in Figure 1, the tolunitrile is preferably cooled by a heat exchanger and stored in an intermediate tank.

<Method for purifying phthalonitrile>

The present invention also provides a method for purifying a phthalonitrile. The purifying method of the present invention includes:

a collection step in which a collection liquid is obtained by bringing a reaction product gas that contains a phthalonitrile and a cyanobenzamide into contact with an organic solvent that has a boiling point lower than that of the phthalonitrile;

a separation step in which the collection liquid is distilled by a high boiling point fraction-separating column, thereby obtaining a gas that contains the phthalonitrile and the organic solvent from the column top, while obtaining a bottom liquid that contains a cyanobenzamide from the column bottom, said bottom liquid having a phthalonitrile content of 90% by mass or less;

a combustion step in which the obtained bottom liquid is subjected to combustion, while being kept in a liquid state; and

a rectification step in which a purified phthalonitrile is obtained by removing the organic solvent from the gas, wherein wherein in the separation step, the liquid residence time at the column bottom is adjusted to 72 hours or less, the distillation pressure is adjusted to 12 kPa or less, and the temperature of the column bottom is adjusted to 200 to 230°C.

The details of the collection step, the separation step, the combustion step and the rectification step are as described with respect to the production method of the present invention.

<Treatment method>

Described herein is also a method for subjecting a treated liquid, which contains 90% by mass or less of a phthalonitrile, a cyanobenzamide and an organic solvent, to combustion, while the treated liquid is kept in a liquid state, at the time of producing a phthalonitrile by means of an ammoxidation reaction (hereinafter sometimes referred to as "the treatment method of the present description"). When the treatment method of the present description is carried out at the time of the production of the phthalonitrile by means of the ammoxidation reaction or the purification, the production or purification can be stably carried out for a long period of time, and preferably, the production or purification can be stably carried out for a long period of time with a reduced loss of isophthalonitrile.

The details of the ammoxidation reaction, the phthalonitrile, the cyanobenzamide and the organic solvent are as described with respect to the production method of the present invention. Further, the details of the treatment are as described in (4) Combustion step with respect to the production method of the present invention.

In one embodiment of the treatment method of the present description, the treated liquid is preferably the bottom liquid generated in the production method or purifying method of the present invention.

The treatment method of the present description preferably comprises: a separation step in which a collection liquid that contains a phthalonitrile, a cyanobenzamide and an organic solvent is distilled by a high boiling point fraction-separating column, thereby obtaining a gas that contains the phthalonitrile and the organic solvent from the column top, while obtaining a bottom liquid that contains a cyanobenzamide from the column bottom, said bottom liquid having a phthalonitrile content of 90% by mass or less; and a combustion step in which the obtained bottom liquid is subjected to combustion, while being kept in a liquid state.

The details of the separation step and the combustion step are as described with respect to the production method of the present invention.

In the separation step, the content of the phthalonitrile in the bottom liquid is preferably 60% by mass or less, more preferably 40% by mass or less, even more preferably 20% by mass or less, particularly preferably 10% by mass or less, and most preferably less than 10% by mass. The lower limit of the content of the phthalonitrile is not particularly limited, but it is preferably 5% by mass or more. Preferred measurement conditions and method are as described with respect to the production method of the present invention.

In the separation step, the distillation pressure of the bottom liquid is preferably 12 kPa or less, more preferably less than 12 kPa, and particularly preferably less than 8 kPa. The lower limit of the distillation pressure is not particularly limited, but it is preferably 5 kPa or more.

In the separation step, the temperature of the bottom liquid is preferably 200 to 230°C, more preferably 210 to 230°C, and particularly preferably 220 to 230°C.

In the separation step, the liquid residence time of the bottom liquid is preferably less than 96 hours, and particularly preferably 72 hours or less. The lower limit of the liquid residence time is not particularly limited and it is sufficient when it is 0 hour or more, but it is preferably 12 hours or more, particularly preferably 24 hours or more, and most preferably 48 hours or more.

In the separation step, regarding the bottom liquid, it is preferred that the distillation pressure is 5 to 12 kPa, that the temperature is 200 to 230°C, and that the liquid residence time is 12 to 72 hours. It is more preferred that the distillation pressure is 5 kPa or more but less than 12 kPa, that the temperature is 210 to 230°C, and that the liquid residence time is 12 to 72 hours. It is particularly preferred that the distillation pressure is 5 kPa or more but less than 8 kPa, that the temperature is 220 to 230°C, and that the liquid residence time is 12 to 72 hours.

EXAMPLES

Hereinafter, the present invention will be described in detail by way of experiment examples, but the present invention is not limited thereto.

[Experiment Example 1]

Using a mixture containing 73.5 wt% of meta-tolunitrile, 25 wt% of isophthalonitrile, 1 wt% of cyanobenzamide (3-cyanobenzamide) and 0.5 wt% of other components, distillation, purification and separation of isophthalonitrile were carried out according to the flow shown in Figure 2.

The above-described mixture was supplied to the middle section of a high boiling point fraction-separating column. Regarding distillation conditions, the column top pressure of the high boiling point fraction-separating column was adjusted to 12 kPa, and the column bottom temperature was adjusted to 200°C. In a bottom liquid, the concentration of isophthalonitrile was 55.7 wt%, the concentration of cyanobenzamide was 26.7 wt%, and the concentration of the other components was 17.6 wt%.

When the bottom liquid was held at 200°C for 72 hours, flowability was maintained.

After that, the bottom liquid was passed through a double pipe (outside passage: steam at about 230°C) using a pump, and injected into a horizontal cylindrical furnace using a nozzle to be burned. There was no closure of the piping, and the bottom liquid was successfully burned.

[Experiment Example 2]

Using a mixture having the same composition as that in Experiment Example 1, distillation, purification and separation of isophthalonitrile were carried out according to the flow shown in Figure 2.

The above-described mixture was supplied to the middle section of a high boiling point fraction-separating column. Regarding distillation conditions, the column top pressure of the high boiling point fraction-separating column was adjusted to 8 kPa, and the column bottom temperature was adjusted to 215°C. In a bottom liquid, the concentration of isophthalonitrile was 35.7 wt%, the concentration of cyanobenzamide was 33.1 wt%, and the concentration of the other components was 31.2 wt%.

When the bottom liquid was held at 215°C for 72 hours, flowability was maintained. After that, the bottom liquid was transferred to an incinerator in a manner similar to that in Experiment Example 1. There was no closure of the piping, and the bottom liquid was successfully burned.

[Experiment Example 3]

Using a mixture having the same composition as that in Experiment Example 1, distillation, purification and separation of isophthalonitrile were carried out according to the flow shown in Figure 2.

The above-described mixture was supplied to the middle section of a high boiling point fraction-separating column. Regarding distillation conditions, the column top pressure of the high boiling point fraction-separating column was adjusted to 5 kPa, and the column bottom temperature was adjusted to 230°C. In a bottom liquid, the concentration of isophthalonitrile was 7.5 wt%, the concentration of cyanobenzamide was 56.7 wt%, and the concentration of the other components was 35.8 wt%.

When the bottom liquid was held at 230°C for 72 hours, flowability was maintained. After that, the bottom liquid was transferred to an incinerator in a manner similar to that in Experiment Example 1. There was no closure of the piping, and the bottom liquid was successfully burned.

[Comparative Example 1]

Using a mixture having the same composition as that in Experiment Example 1, distillation, purification and separation of isophthalonitrile were carried out according to the flow shown in Figure 2.

The above-described mixture was supplied to the middle section of a high boiling point fraction-separating column. Regarding distillation conditions, the column top pressure of the high boiling point fraction-separating column was adjusted to 12 kPa, and the column bottom temperature was adjusted to 200°C. In a bottom liquid, the concentration of isophthalonitrile was 55.7 wt%, the concentration of cyanobenzamide was 26.7 wt%, and the concentration of the other components was 17.6 wt%.

When the bottom liquid was held at 200°C for 96 hours, flowability was lost. Transferring to an incinerator was tried in a manner similar to that in Experiment Example 1, but it was impossible.

[Comparative Example 2]

Using a mixture having the same composition as that in Experiment Example 1, distillation, purification and separation of isophthalonitrile were carried out according to the flow shown in Figure 2.

The above-described mixture was supplied to the middle section of a high boiling point fraction-separating column. Regarding distillation conditions, the column top pressure of the high boiling point fraction-separating column was adjusted to 8 kPa, and the column bottom temperature was adjusted to 215°C. In a bottom liquid, the concentration of isophthalonitrile was 35.7 wt%, the concentration of cyanobenzamide was 33.1 wt%, and the concentration of the other components was 31.2 wt%.

When the bottom liquid was held at 215°C for 96 hours, flowability was lost. Transferring to an incinerator was tried in a manner similar to that in Experiment Example 1, but it was impossible.

[Comparative Example 3]

Using a mixture having the same composition as that in Experiment Example 1, distillation, purification and separation of isophthalonitrile were carried out according to the flow shown in Figure 2.

The above-described mixture was supplied to the middle section of a high boiling point fraction-separating column. Regarding distillation conditions, the column top pressure of the high boiling point fraction-separating column was adjusted to 5 kPa, and the column bottom temperature was adjusted to 230°C. In a bottom liquid in the high boiling point fraction-separating column, the concentration of isophthalonitrile was 7.5 wt%, the concentration of cyanobenzamide was 56.7 wt%, and the concentration of the other components was 35.8 wt%.

When the bottom liquid was held at 230°C for 96 hours, flowability was lost. Transferring to an incinerator was tried in a manner similar to that in Experiment Example 1, but it was impossible.

In Experiment Examples 1-3, flowability of the bottom liquid was maintained after the bottom liquid was held. This indicates that the melting point of the bottom liquid was lower than the temperature of the bottom liquid. The bottom liquid whose flowability is maintained can be transferred to an incinerator. As a result, closure of the piping can be avoided, the phthalonitrile can be stably produced for a long period of time, and the phthalonitrile can be stably purified for a long period of time.

Meanwhile, in Comparative Examples 1-3, flowability of the bottom liquid was lost after the bottom liquid was held. This indicates that the melting point of the bottom liquid was higher than the temperature of the bottom liquid. As a result, closure of the piping was caused, and it was impossible to transfer the bottom liquid to the incinerator.

The compositions and melting points of the bottom liquid before and after holding in Experiment Examples 1-3 and Comparative Examples 1-3 are shown in Table 1 below.

The compositions of the bottom liquid before and after holding were measured by means of gas chromatography. Specifically, the bottom liquid was sampled and cooled to room temperature, and after that, it was pulverized, dissolved in a solvent, and then analyzed by means of gas chromatography. As a measurement apparatus, Shimadzu GC-2025 manufactured by Shimadzu Corporation was used. As a detection unit, Flame Ionization Detector (FID) was used, and as a carrier gas, helium gas was used.

Table 1

| | Column bottom temperature | Pressure | Time | Isophthalonitrile | | Cyanobenzamide | | Others | | Melting point |
|---|---|---|---|---|---|---|---|---|---|---|
| | °C | Kpa | Hr | Content rate (Wt%) | Decrease rate (%) | Content rate (wt%) | Decrease rate (%) | Content rate (wt%) | Increase rate (%) | °C |
| Experiment Example 1 | 200 | 12 | 0 | 55.7 | 73.1 | 26.7 | 23.2 | 17.6 | 266.5 | 200 or lower |
| | | | 72 | 15.0 | | 20.5 | | 64.5 | | 200 or lower |
| Experiment Example 2 | 215 | 8 | 0 | 35.7 | 72.0 | 33.1 | 16.9 | 31.2 | 100.3 | 215 or lower |
| | | | 72 | 10.0 | | 27.5 | | 62.5 | | 215 or lower |
| Experiment Example 3 | 230 | 5 | 0 | 7.5 | 32.0 | 56.7 | 46.7 | 35.8 | 80.7 | 230 or lower |
| | | | 72 | 5.1 | | 30.2 | | 64.7 | | 230 or lower |
| Comparative Example 1 | 200 | 12 | 0 | 55.7 | 88.7 | 26.7 | 43.8 | 17.6 | 347.2 | 200 or higher |
| | | | 96 | 6.3 | | 15.0 | | 78.7 | | 200 or higher |
| Comparative Example 2 | 215 | 8 | 0 | 35.7 | 88.0 | 33.1 | 40.2 | 31.2 | 143.3 | 215 or higher |
| | | | 96 | 4.3 | | 19.8 | | 75.9 | | 215 or higher |
| Comparative Example 3 | 230 | 5 | 0 | 7.5 | 62.7 | 56.7 | 64.4 | 35.8 | 115.1 | 230 or higher |
| | | | 96 | 2.8 | | 20.2 | | 77.0 | | 230 or higher |

It is understood from Table 1 that change in quality of the bottom liquids of Experiment Examples 1-3 due to holding was suppressed more when compared to the bottom liquids of Comparative Examples 1-3.

[Reference Example 1]

Using a mixture having the same composition as that in Experiment Example 1, distillation, purification and separation of isophthalonitrile were carried out according to the flow shown in Figure 2.
The above-described mixture was supplied to the middle section of a high boiling point fraction-separating column. Regarding distillation conditions, the column top pressure of the high boiling point fraction-separating column was adjusted to 8 kPa, and the column bottom temperature was adjusted to 215°C. In a bottom liquid, the concentration of isophthalonitrile was 35.7 wt%, the concentration of cyanobenzamide was 33.1 wt%, and the concentration of the other components was 31.2 wt%.
When the bottom liquid was held at 215°C for 72 hours, flowability was maintained, but when the bottom liquid was directly drawn out to the outside of the system, the high boiling point liquid was solidified and it became difficult to handle it.

[Reference Example 2]

Using a mixture having the same composition as that in Experiment Example 1, distillation, purification and separation of isophthalonitrile were carried out according to the flow shown in Figure 2.
The above-described mixture was supplied to the middle section of a high boiling point fraction-separating column. Regarding distillation conditions, the column top pressure of the high boiling point fraction-separating column was adjusted to 8 kPa, and the column bottom temperature was adjusted to 215°C. In a bottom liquid, the concentration of isophthalonitrile was 35.7 wt%, the concentration of cyanobenzamide was 33.1 wt%, and the concentration of the other components was 31.2 wt%.
At the column bottom at 215°C, flowability of the high boiling point liquid was maintained. When the bottom liquid was quickly drawn out to the outside of the system, the high boiling point liquid was solidified and it became difficult to handle it.
When obtaining the purified phthalonitrile by distillation of the phthalonitrile containing the tolunitrile as the main low boiling point impurity and the cyanobenzamide as the main high boiling point impurity, solidification of the bottom liquid in the distillation column, closure, etc. were successfully prevented. Therefore, according to the method of the present invention, a high-quality isophthalonitrile can be stably obtained for a long period of time. Accordingly, by using the method of the present invention, a phthalonitrile can be industrially advantageously produced, and the present invention has great industrial significance.

EXPLANATIONS OF LETTERS OR NUMERALS

A: ammoxidation reactor
B: collection column
C: high boiling point fraction-separating column
D: rectification column

**Claims**

1.   A method for producing a purified phthalonitrile, which comprises:

a production reaction step for obtaining a reaction product gas comprising a phthalonitrile and a cyanobenzamide by reacting ammonia, oxygen and xylene in the presence of a catalyst;
a collection step for obtaining a collection liquid by bringing the reaction product gas into contact with an organic solvent that has a boiling point lower than that of the phthalonitrile;
a separation step for distilling the collection liquid by a high boiling point fraction-separating column, thereby obtaining a gas that comprises the phthalonitrile and the organic solvent from the column top, while obtaining a bottom liquid that comprises a cyanobenzamide from the column bottom, said bottom liquid having a phthalonitrile content of 90% by mass or less;
a combustion step for subjecting the obtained bottom liquid to combustion, while being kept in a liquid state; and

a rectification step for obtaining a purified phthalonitrile by removing the organic solvent from the gas obtained from the column top,
wherein in the separation step, the liquid residence time at the column bottom is adjusted to 72 hours or less, the distillation pressure is adjusted to 12 kPa or less, and the temperature of the column bottom is adjusted to 200 to 230°C.

2. The method for producing a purified phthalonitrile according to claim 1, wherein:

the steps from the production reaction step to the rectification step are continuously carried out;
in the production reaction step, the reaction product gas further contains a tolunitrile; and
the tolunitrile is recovered and used as the organic solvent in the collection step.

3. The method for producing a purified phthalonitrile according to claim 2, wherein the tolunitrile is meta-tolunitrile, para-tolunitrile or a mixture thereof.

4. The method for producing a purified phthalonitrile according to any one of claims 1 to 3, wherein the cyanobenzamide is 3-cyanobenzamide, 4-cyanobenzamide or a mixture thereof.

5. The method for producing a purified phthalonitrile according to any one of claims 1 to 4, wherein the phthalonitrile is isophthalonitrile, terephthalonitrile or a mixture thereof.

6. A method for purifying a phthalonitrile, which comprises:

a collection step for obtaining a collection liquid by bringing a reaction product gas that comprises a phthalonitrile and a cyanobenzamide into contact with an organic solvent that has a boiling point lower than that of the phthalonitrile;
a separation step for distilling the collection liquid by a high boiling point fraction-separating column, thereby obtaining a gas that comprises the phthalonitrile and the organic solvent from the column top, while obtaining a bottom liquid that comprises a cyanobenzamide from the column bottom, said bottom liquid having a phthalonitrile content of 90% by mass or less;
a combustion step for subjecting the obtained bottom liquid to combustion, while being kept in a liquid state; and
a rectification step for obtaining a purified phthalonitrile by removing the organic solvent from the gas obtained from the column top,
wherein in the separation step, the liquid residence time at the column bottom is adjusted to 72 hours or less, the distillation pressure is adjusted to 12 kPa or less, and the temperature of the column bottom is adjusted to 200 to 230°C.

7. The method for producing a purified phthalonitrile according to any one of claims 1 to 5, wherein the combustion step further comprises generating steam by utilizing heat generated by combustion.

8. The method for producing a purified phthalonitrile according to claim 7, wherein the combustion step further comprises transferring the bottom liquid by using a piping, and wherein the temperature of the piping is kept by using the steam.

9. The method for producing a purified phthalonitrile according to claim 7 or 8, wherein the steam is used as a heat source in the collection step, the separation step or the rectification step.


**Patentansprüche**

1. Verfahren zur Herstellung eines gereinigten Phthalonitrils, das umfasst:

einen Herstellungsreaktionsschritt zum Erhalten eines Reaktionsproduktgases, umfassend ein Phthalonitril und ein Cyanobenzamid, durch Reaktion von Ammoniak, Sauerstoff und Xylol in Gegenwart eines Katalysators,
einen Sammelschritt zum Erhalten einer Sammelflüssigkeit durch Inkontaktbringen des Reaktionsproduktgases mit einem organischen Lösungsmittel, das einen niedrigeren Siedepunkt als der des Phthalonitrils aufweist,
einen Trennschritt zum Destillieren der Sammelflüssigkeit durch eine Fraktion mit hohem Siedepunkt abtrennende Kolonne, wodurch ein Gas, das das Phthalonitril und das organische Lösungsmittel umfasst, von dem Kolonnenkopf erhalten wird, während eine Bodenflüssigkeit, die ein Cyanobenzamid umfasst, vom Kolonnenboden erhalten wird, wobei die Bodenflüssigkeit einen Phthalonitrilgehalt von 90 Masse-% oder weniger aufweist,

einen Verbrennungsschritt zum Unterziehen der erhaltenen Bodenflüssigkeit einer Verbrennung, während sie in einem flüssigen Zustand gehalten wird, und

einen Rektifikationsschritt zum Erhalten eines gereinigten Phthalonitrils durch Entfernung des organischen Lösungsmittels von dem vom Kolonnenkopf erhaltenen Gas,

wobei in dem Trennschritt die Flüssigkeitsverweilzeit am Kolonnenboden auf 72 Stunden oder weniger eingestellt wird, der Destillationsdruck auf 12 kPa oder weniger eingestellt wird und die Temperatur am Kolonnenboden auf 200 bis 230°C eingestellt wird.

2. Verfahren zur Herstellung eines gereinigten Phthalonitrils gemäß Anspruch 1, wobei

die Schritte vom Herstellungsreaktionsschritt bis zum Rektifikationsschritt kontinuierlich durchgeführt werden, im Herstellungsreaktionsschritt das Herstellungsproduktgas ferner ein Tolunitril enthält und

das Tolunitril zurückgewonnen und als das organische Lösungsmittel im Sammelschritt verwendet wird.

3. Verfahren zur Herstellung eines gereinigten Phthalonitrils gemäß Anspruch 2, wobei das Tolunitril meta-Tolunitril, para-Tolunitril oder eine Mischung davon ist.

4. Verfahren zur Herstellung eines gereinigten Phthalonitrils gemäß einem der Ansprüche 1 bis 3, wobei das Cyanobenzamid 3-Cyanobenzamid, 4-Cyanobenzamid oder eine Mischung davon ist.

5. Verfahren zur Herstellung eines gereinigten Phthalonitrils gemäß einem der Ansprüche 1 bis 4, wobei das Phthalonitril Isophthalonitril, Terephthalonitril oder eine Mischung davon ist.

6. Verfahren zur Reinigung eines Phthalonitrils, das umfasst:

einen Sammelschritt zum Erhalten einer Sammelflüssigkeit durch Inkontaktbringen eines Reaktionsproduktgases, das ein Phthalonitril und ein Cyanobenzamid umfasst, mit einem organischen Lösungsmittel, das einen niedrigeren Siedepunkt als der des Phthalonitrils aufweist,

einen Trennschritt zum Destillieren der Sammelflüssigkeit durch eine eine Fraktion mit hohem Siedepunkt abtrennende Kolonne, wodurch ein Gas, das das Phthalonitril und das organische Lösungsmittel enthält, von dem Kolonnenkopf erhalten wird, während eine Bodenflüssigkeit, die ein Cyanobenzamid umfasst, von dem Kolonnenboden erhalten wird, wobei die Bodenflüssigkeit einen Phthalonitrilgehalt von 90 Masse-% oder weniger aufweist,

einen Verbrennungsschritt zum Unterziehen der erhaltenen Bodenflüssigkeit einer Verbrennung, während sie in einem flüssigen Zustand gehalten wird, und

einen Rektifikationsschritt zum Erhalten eines gereinigten Phthalonitrils durch Entfernung des organischen Lösungsmittels von dem vom Kolonnenkopf erhaltenen Gas,

wobei im Trennschritt die Flüssigkeitsverweilzeit am Kolonnenboden auf 72 Stunden oder weniger eingestellt wird, der Destillationsdruck auf 12 kPa oder weniger eingestellt wird und die Temperatur am Kolonnenboden auf 200 bis 230°C eingestellt wird.

7. Verfahren zur Herstellung eines gereinigten Phthalonitrils gemäß einem der Ansprüche 1 bis 5, wobei der Verbrennungsschritt die Erzeugung von Dampf durch Ausnutzung der durch die Verbrennung erzeugten Wärme umfasst.

8. Verfahren zur Herstellung eines gereinigten Phthalonitrils gemäß Anspruch 7, wobei der Verbrennungsschritt ferner das Überführen der Bodenflüssigkeit durch Verwendung einer Rohrleitung umfasst und wobei die Temperatur in der Rohrleitung unter Verwendung des Dampf aufrechterhalten wird.

9. Verfahren zur Herstellung eines gereinigten Phthalonitrils gemäß Anspruch 7 oder 8, wobei der Dampf als eine Wärmequelle im Sammelschritt, Trennschritt oder Rektifikationsschritt verwendet wird.

**Revendications**

1. Procédé de production d'un phtalonitrile purifié, qui comprend :

une étape de réaction de production pour obtenir un gaz de produit de réaction comprenant un phtalonitrile et un cyanobenzamide par réaction d'ammoniac, d'oxygène et de xylène en présence d'un catalyseur ;

une étape de collecte pour obtenir un liquide de collecte en amenant le gaz de produit de réaction en contact avec un solvant organique qui présente un point d'ébullition inférieur à celui du phtalonitrile ;

une étape de séparation pour distiller le liquide de collecte par une colonne de séparation de fraction à point d'ébullition élevé, obtenant ainsi un gaz qui comprend le phtalonitrile et le solvant organique depuis le sommet de colonne, tout en obtenant un liquide de fond qui comprend un cyanobenzamide depuis le fond de colonne, ledit liquide de fond présentant une teneur en phtalonitrile de 90 % en masse ou moins ;

une étape de combustion pour soumettre le liquide de fond obtenu à une combustion, tout en étant maintenu dans un état liquide ; et

une étape de rectification pour obtenir un phtalonitrile purifié en éliminant le solvant organique du gaz obtenu depuis le sommet de colonne,

dans lequel à l'étape de séparation, le temps de résidence de liquide au niveau de fond de colonne est réglé sur 72 heures ou moins, la pression de distillation est réglée sur 12 kPa ou moins, et la température du fond de colonne est réglée sur 200 à 230 °C.

2. Procédé de production d'un phtalonitrile purifié selon la revendication 1, dans lequel :

les étapes allant de l'étape de réaction de production à l'étape de rectification sont mises en oeuvre de manière continue ;

à l'étape de réaction de production, le gaz de produit de réaction contient en outre un tolunitrile ; et

le tolunitrile est récupéré et utilisé en tant que solvant organique à l'étape de collecte.

3. Procédé de production d'un phtalonitrile purifié selon la revendication 2, dans lequel le tolunitrile est du méta-tolunitrile, du para-tolunitrile ou un mélange de ceux-ci.

4. Procédé de production d'un phtalonitrile purifié selon l'une quelconque des revendications 1 à 3, dans lequel le cyanobenzamide est du 3-cyanobenzamide, du 4-cyanobenzamide ou un mélange de ceux-ci.

5. Procédé de production d'un phtalonitrile purifié selon l'une quelconque des revendications 1 à 4, dans lequel le phtalonitrile est de l'isophthalonitrile, du térephtalonitrile ou un mélange de ceux-ci.

6. Procédé de purification d'un phtalonitrile, qui comprend :

une étape de collecte pour obtenir un liquide de collecte en amenant un gaz de produit de réaction qui comprend un phtalonitrile et un cyanobenzamide en contact avec un solvant organique qui présente un point d'ébullition inférieur à celui du phtalonitrile ;

une étape de séparation pour distiller le liquide de collecte par une colonne de séparation de fraction à point d'ébullition élevé, obtenant ainsi un gaz qui comprend le phtalonitrile et le solvant organique depuis le sommet de colonne, tout en obtenant un liquide de fond qui comprend un cyanobenzamide depuis le fond de colonne, ledit liquide de fond présentant une teneur en phtalonitrile de 90 % en masse ou moins ;

une étape de combustion pour soumettre le liquide de fond obtenu à une combustion, tout en étant maintenu dans un état liquide ; et

une étape de rectification pour obtenir un phtalonitrile purifié en éliminant le solvant organique du gaz obtenu depuis le sommet de colonne,

dans lequel à l'étape de séparation, le temps de résidence de liquide au niveau de fond de colonne est réglé sur 72 heures ou moins, la pression de distillation est réglée sur 12 kPa ou moins, et la température du fond de colonne est réglée sur 200 à 230 °C.

7. Procédé de production d'un phtalonitrile purifié selon l'une quelconque des revendications 1 à 5, dans lequel l'étape de combustion comprend en outre la génération de vapeur en utilisant la chaleur générée par combustion.

8. Procédé de production d'un phtalonitrile purifié selon la revendication 7, dans lequel l'étape de combustion comprend en outre le transfert du liquide de fond en utilisant une conduite, et dans lequel la température de la conduite est maintenue en utilisant la vapeur.

9. Procédé de production d'un phtalonitrile purifié selon la revendication 7 ou la revendication 8, dans lequel la vapeur est utilisée comme source de chaleur à l'étape de collecte, l'étape de séparation ou l'étape de rectification.

[Figure 1]

(1) Production reaction step　　(2) Collection step　　　　(3) Separation step　(5) Rectification step

(4) Combustion step

[Figure 2]

[Figure 3]

(1) Production reaction step    (2) Collection step    (3) Separation step    (5) Rectification step

(4) Combustion step

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4929523 B
- US 2002035287 A1
- JP H11209332 A
- JP H0971561 A

**Non-patent literature cited in the description**

- Process Handbook. MGC-Badger Isophthalonitril Process, 1976